# EUROPEAN PATENT SPECIFICATION

(11) **EP 0 975 601 B1**
(45) Date of publication and mention of the grant of the patent: **12.03.2003**
(21) Application number: 98919219.0
(22) Date of filing: 03.04.1998
(51) Int. Cl.: C07D 217/26, C07C 321/28, C07C 319/14, C07D 303/36

(54) **PROCESS FOR MAKING A BUTYLTHIO-ISOQUINOLINE AND INTERMEDIATES THEREFOR**
VERFAHREN ZUR HERSTELLUNG EINES BUTHYLTHUIO-ISOCHINOLINDERIVATES UND ZWISCHENPRODUKTE DAFÜR
PROCEDE SERVANT A PREPARER UNE BUTYLTHIO-ISOQUINOLEINE ET SES INTERMEDIAIRES

(30) Priority: 10.04.1997 EP 97810212; 18.04.1997 EP 97810240
(43) Date of publication of application: 02.02.2000
(73) Proprietor: F. HOFFMANN-LA ROCHE AG, 4070 Basel (CH)
(72) Inventor: HILPERT, Hans, CH-4153 Reinach (CH)
(74) Representative: Rauber, Beat
(86) International application number: EP9801959
(87) International publication number: WO98045271

(56) References cited:
- WO-A-97/11938
- US-A- 5 484 926
- US-A- 5 705 647

## Description

The present invention is concerned with a novel process for the manufacture of a phenylthiobutyl-isoquinoline of the formula

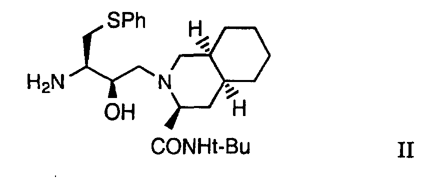

The compound of formula II and its preparation starting from L-serine is described, e.g. in USP 5.484.926. This compound is a valuable intermediate for the manufacture of pharmacologically active compounds, suitable for the treatment of viral infections, especially those caused by HIV and other retroviruses, described in the mentioned US patent, e.g. at columns 16 and 17, such as represented by formula 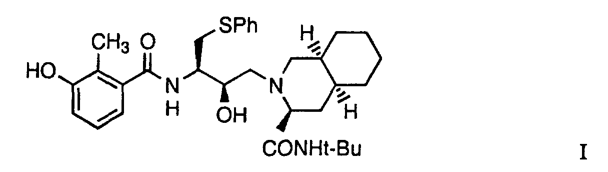 wherein Ph is phenyl,
and of pharmaceutically acceptable salts thereof.

Alternative processes for producing the compound the compound of formula II are described in WO 97/11938 and in US-A 5 705 647.

The process of the present invention is characterized by less reaction steps, more convenient reaction conditions and a higher overall yield of the desired stereoisomer of formula II.

The process of the present invention comprises
(a) reacting diprotected L-serine of formula 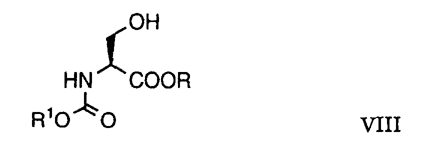 wherein R is lower alkyl and R¹ is lower alkyl or benzyl, with mesyl or tosyl chloride and a thiophenolate;
(b) reacting the resulting phenylthio compound of formula 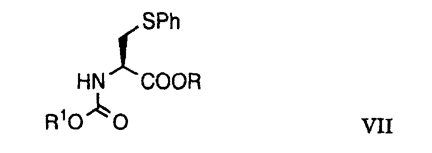 with halogenated methyllithium;
(c) reducing a resulting halogen ketone of formula 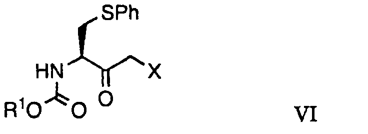 wherein X is halogen,
   to the corresponding halogen alcohol of formula 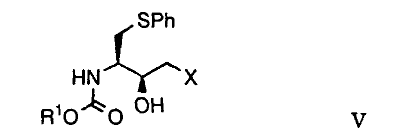
(d) treating the halogen alcohol of formula V with a base to form the [(R)-1-[(S)-oxiran-2-yl]-2-phenylthio-ethyl]-carbamic acid ester of formula 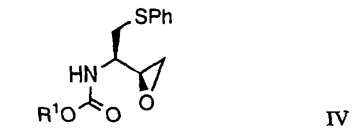
(e) reacting the carbamic acid ester of formula IV with N-tert.-butyl-decahydro-(4aS, 8aS)-isoquinoline-3(S)-carboxamide of formula 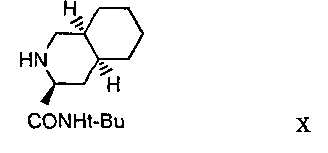 and
(f) treating the resulting compound of formula 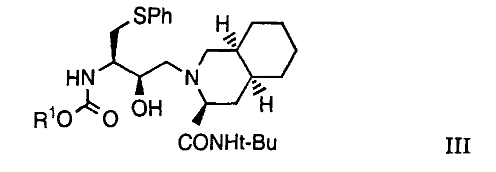 with a base to yield the compound of formula II above.

The term "lower-alkyl" used throughout the specification refers to straight- or branched-chain saturated hydrocarbon residues with 1-6, preferably 1-4, carbon atoms, such as methyl, ethyl, n-propyl, isopropyl, n-butyl, sec.-butyl, tert.-butyl, a pentyl or a hexyl group with methyl and ethyl being preferred, especially for R¹. Especially preferred in connection with the present invention are compounds wherein R¹ is methyl. Halogen denotes chlorine, bromine and iodine with chlorine being preferred.

The starting diprotected L-serines of formula VIII are known compounds and can easily be prepared from L-serine via the corresponding L-serine lower alkyl esters with the corresponding chloroformates.

The phenylthio compounds of formula VII may be prepared using methods known in the art (e.g. Sasaki et al., Tetrahedron Letters 28, 6069 [1987]). The amino- and carboxy-protected L-serine is transformed into its tosylate or mesylate in the presence of an amine such as pyridine or triethylamine in an aprotic solvent such as methylene chloride or acetic acid ester and then reacted with a thiophenolate, if desired prepared in situ from thiophenol and a strong base, at low temperature, preferably a temperature from -10°C up to 0°C.

The halomethylation of the resulting phenylthio compound VII is preferably effected using halogenated methyllithium which is generated in situ. The latter is conveniently formed using dihalogenated methane, e.g., dichloro-, dibromo- or diiodomethane, preferably using bromochloromethane, and a lower-alkyl-lithium, preferably butyllithium or hexyllithium, in an ether, preferably tetrahydrofuran, at -20° to -120°C, preferably -80°C.

The halomethylation of the phenylthio compound VII to the halogen ketone VI can conveniently be carried out by
(a) reacting the compound VII with a lower-alkyl-lithium and an organochlorosilane of the formula ClSi(R², R³, R⁴), wherein R², R³ and R⁴ are lower-alkyl or phenyl, and
(b) treating the silyl-protected compound of general formulae VII-A and/or VII-B formed as an intermediate 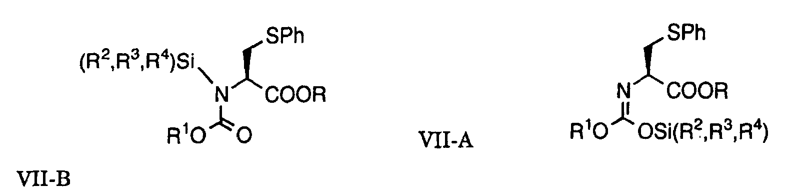 in the presence of dihalogenated methane and a lower-alkyl-lithium.

Surprisingly the previous protection of the carbamate group present in compound VII by a silyl group yielding compounds VII-A and/or VII-B as intermediates leads to a considerable increase in yield. Butyllithium or hexyllithium is preferably used as the lower-alkyl-lithium and chlorotrimethylsilane is preferably used as the organochlorosilane ClSi(R²,R³,R⁴). Moreover, an almost complete halomethylation can be achieved using significantly less lower-alkyl-lithium and dihalogenated methane.

The reduction of the halogen ketone VI to the corresponding alcohol V is conveniently carried out with a hydride in a solvent such as toluene, tetrahydrofuran or an alcohol, preferably methanol, ethanol or isopropanol, at a temperature between -30 and 80°C, preferably between -15°C and 50°C, optionally under reduced pressure, using sodium bis-(2-methoxy-ethoxy)-aluminium hydride, lithium aluminium hydride, lithium aluminium tri-tert.-butoxyhydride, sodium borohydride, tetramethylammonium borohydride or, preferably, using an aluminium tri-alkoxide or lithium aluminium trialkoxyhydride. The term "alkoxide" means lower alkoxy with the lower-alkyl residue being as defined above, comprising e.g. methoxy, ethoxy, n-propoxy, isopropoxy, n-butoxy, isobutoxy, tert.-butoxy, as well as pentyloxy or hexyloxy groups. The aluminium compounds can have identical or different alkoxy groups. Aluminium tri-isopropoxide and aluminium tri-sec.-butoxide are especially preferred compounds. The reagents lithium aluminium tri-tert.-butoxyhydride, aluminium tri-isopropoxide and aluminium tri-sec.-butoxide gave unexpected high stereoselectivity in a molar ratio of at least 9:1 of the (1S,2S) and (1S, 2R) isomeric halohydrins V, which could be crystallized in >99% optical purity and with high yield.

The ring closure of the halohydrin of formula V to form the corresponding epoxide of formula IV is conveniently carried out in a solvent such as ethanol or preferably a toluene/water mixture in the presence of a base such as an alkaline or alkaline earth metal hydroxide, preferably sodium or potassium hydroxide, at a temperature between 0° and 80°C, preferably 40-50°C. The epoxide which is formed need not be purified.

The reaction of the epoxide of formula IV with the isoquinoline X to form compound III is conveniently carried out in a solvent such as a hydrocarbon, e.g. toluene, or a lower-alkanol, preferably ethanol, while heating under reflux, preferably 20-100°C, most preferably 80°C.

The cleavage of the N-protecting group from the compound of formula III is carried out in a manner known per se, conveniently in a solvent such as water, ethanol or a mixture thereof, using a base such as sodium or potassium hydroxide while heating to the reflux temperature, preferably 20-100°C, especially 80°C.

The intermediate compounds of formulae VII, VI, V, IV and III wherein R¹ is lower alkyl, viz. compounds of formulae VII-1, VI-1, V-1, IV-1 and III-1, are new and a further aspect of the present invention as well as their use in the preparation of the compound of formula II or the pharmacologically active compounds, such as the compound of formula I and their salts mentioned above. A preferred embodiment of the new compounds are those wherein R¹ is C₁₋₃ alkyl, especially methyl or ethyl.

Especially preferred compounds of formulae III, IV, V, VI and VII are those wherein R and R¹ are methyl and X is chlorine, viz.
methyl (1R, 2R)-[1-phenylthiomethyl-3-[(3S, 4aS, 8aS)-3-tert.-butoxycarbamoyl-decahydroisoquinol-2-yl]-2-hydroxypropyl]-carbamate;
methyl [(R)-1-[(S)-oxiran-2-yl]-2-phenylthio-ethyl]-carbamate;
methyl (1R, 2S)-[3-chloro-2-hydroxy-1-(phenylthiomethyl)-propyl]-carbamate;
methyl [3-chloro-2-oxo-(R)-1-(phenylthiomethyl)-propyl]-carbamate and
methyl (3-phenylthio-(R)-2-methoxycarbonylamino)-propionate.

Finally, the compounds of formulae II, III, IV, V, VI and VII, when obtained by the process described hereinbefore or in the following Examples, are a further aspect of the present invention.

A compound of formula I can be obtained, e.g., in accordance with the method described in Example 23 of USP 5.484.926, using 70mg of the compound of formula II, 24.6mg of 3-hydroxy-2-methylbenzoic acid, 33mg of DDC and 22mg of HOBT·H₂O in 4ml of THF.

The formation of pharmaceutically acceptable salts can be performed in a manner known per se. The methanesulfonic acid salt, e.g., can be prepared as described in Example 75 of USP 5.484.926 by dissolving 3.34g of compound I in 30ml of MeOH and 30ml of methylene chloride and adding a solution of 596mg of methanesulfonic acid in 10ml of methylene chloride dropwise and working up of the reaction mixture to obtain the desired compound in pure form.

Reaction Scheme I summarizes the preferred reaction steps for the preparation of compounds VIII to I wherein R and R¹ are methyl and X is chlorine described in more detail in the specification.

The following Examples illustrate the present invention in more detail.

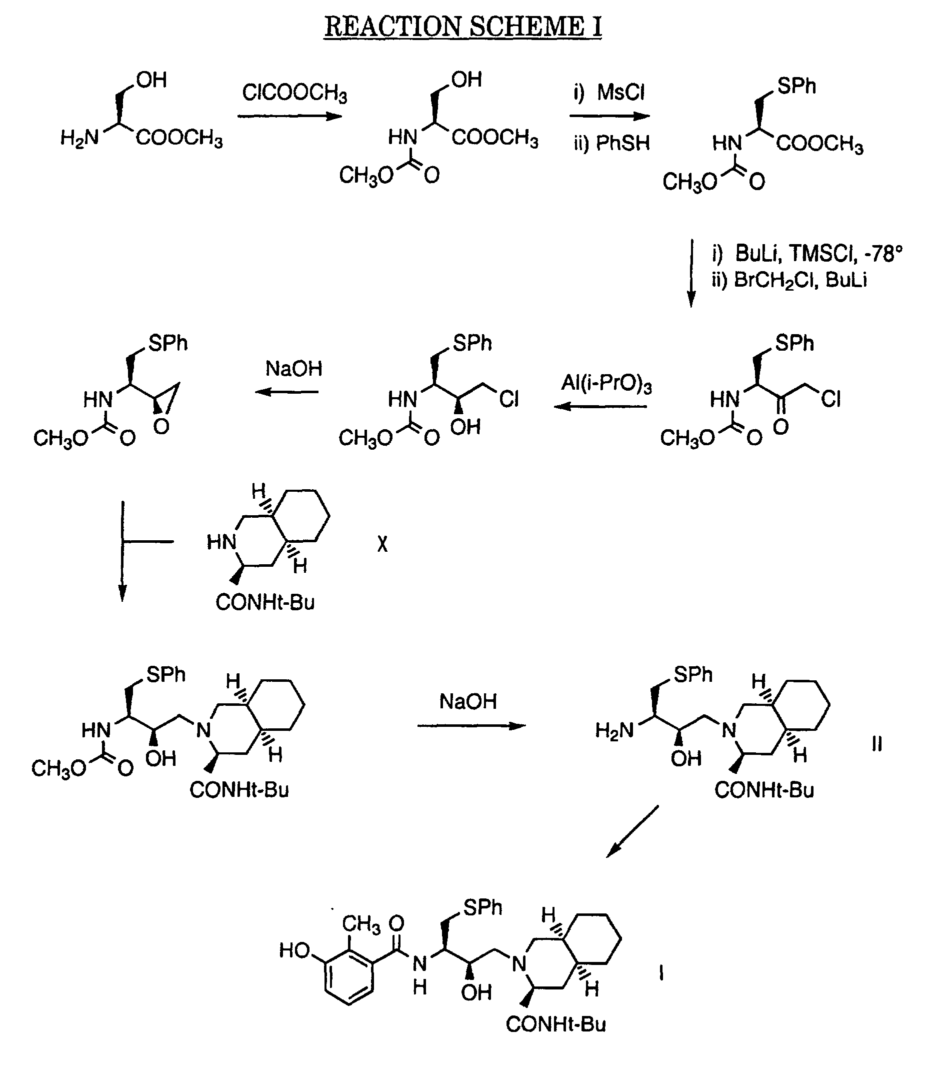

### Example 1

To a solution of 72.03g L-serine-methylester hydrochloride in 400 ml of water were added at 0°C 49.5g of methyl chloroformate. The pH was kept at 6-7 by adding a 40% aqueous solution of sodium hydroxide. After 2 1/2 h at 0°C the mixture was extracted with 6 portions of ethyl acetate, the combined extracts were dried and evaporated to give 75.39g (92%) of pure methyl 3-hydroxy-(S)-2-methoxycarbonylamino-propionate. IR (neat): 3380m (NH, OH), 1720s, br. (C=O), 1535s (amide II). MS (EI): 178/20 (M+H⁺).

### Example 2

To a solution of 8.86g of methyl 3-hydroxy-(S)-2-methoxycarbonylamino-propionate and 5.73g of methanesulfonyl chloride in 80ml of ethyl acetate were added at -10°C 5.06g of triethylamine. After 1 hour at -10°C the mixture was washed with diluted aqueous hydrochloric acid (1N) and water, the ethyl acetate was evaporated and the residue was dissolved in 15 ml of dimethylformamide. The solution was treated at -20°C with a solution of 7.41g of sodium thiophenolate in 30 ml of dimethylformamide and stirring was continued at -20°C for 5 hours. The mixture was diluted with water and extracted with toluene and the toluene was evaporated to give 13.22g (98%) of pure methyl (3-phenylthio-(R)-2-methoxycarbonylamino)-propionate. IR (neat): 3350w (NH), 1745s and 1702s (C=O), 1513s (amide II). MS (EI): 269/20(M⁺).

### Example 3

To a solution of 8.08g of methyl (3-phenylthio-(R)-2-methoxycarbonyl-amino)- propionate in 75ml of tetrahydrofuran were added at -80°C 18ml of a 1.67M solution of butyllithium in hexane followed by addition of 3.58g of chlorotrimethylsilane. The suspension was treated with 4.66g of bromochloromethane followed by addition of 27ml of a 1.67M solution of butyllithium in hexane at -80°C. The solution was quenched with 50ml of 6% aqueous hydrochloric acid and warmed to 22°C. The layers were separated, the organic layer was washed with brine, dried and evaporated to give 8.70g approx. 70% pure methyl [3-chloro-2-oxo-(R)-1-(phenylthiomethyl)-propyl]-carbamate. A sample was crystallized from tetrahydrofuran/hexane, m.p. 91-92.5°C. IR (KBr): 3321s (NH), 1740s and 1681s (C=O), 1539s (amide II). MS (EI): 287/15 (M⁺).

### Example 4

To a suspension of 2.15g aluminium isopropoxide in 35ml of isopropanol were added 2.88g of methyl [3-chloro-2-oxo-(R)-1-(phenylthiomethyl)-propyl]-carbamate and the suspension was stirred at 70°C/ 400mbar for 4 hours. The mixture was cooled to 0°C, the pH was adjusted to 1 by adding hydrochloric acid and the isopropanol was evaporated. The suspension was filtered and the residue was recrystallized from toluene to give 2.18g (75%) isomerically pure methyl (1R, 2S)-[3-chloro-2-hydroxy-1-(phenylthiomethyl)-propyl]-carbamate, m.p. 113-114°C. IR (KBr): 3389m and 3327m (NH, OH), 1695s (C=O), 1537s (amide II). MS (EI): 289/35 (M⁺).

### Example 5

A mixture of 2.90g of methyl (1R, 2S)-[3-chloro-2-hydroxy-1-(phenylthiomethyl)-propyl]-carbamate, 0.8g sodium hydroxide, 20ml toluene and 10ml of water was stirred at 40°C for 2 hours. The organic layer was washed with water and the solvent evaporated. The residue containing methyl [(R)-1-[(S)-oxiran-2-yl]-2-phenylthio-ethyl]-carbamate was mixed with 12ml of ethanol and 2.38g of decahydro-(4aS, 8aS)-isoquinoline-3(S)-N-tert.-butyl-carboxamide and heated at reflux temperature for 5-10 hours. The solvent was evaporated and the residue partitioned between water and dichloromethane. The organic layer was evaporated to give 4.4g (90%) of methyl (1R, 2R)-[1-phenylthiomethyl-3-[(3S, 4aS, 8aS)-3-tert.-butoxycarbamoyl-decahydro-isoquinol-2-yl]-2-hydroxypropyl]-carbamate. IR (KBr): 3410m and 3320m (NH, OH), 1715s and 1660s (C=O), 1550s (amide II).

### Example 6

A suspension of 4.92g of methyl (1R, 2R)-[1-phenyl-thiomethyl-3-[(3S, 4aS, 8aS)-3-tert.-butoxycarbamoyl-decahydroisoquinol-2-yl]-2-hydroxy-propyl]-carbamate and 2.69g of sodium hydroxide in 10ml of ethanol and 10ml of water was heated at reflux for 3-5 hours. The solvent was evaporated and the residue was partitioned between water and dichloromethane. The organic layer was washed with water, dried and evaporated to give 3.9g (90%) 2-[3(R)-amino-2(R)-hydroxy-4-phenylthio-butyl]-decahydro-(4aS, 8aS)-isoquinoline-3(S)-N-tert.-butylcarboxamide.

### Example 7

To a solution of 24.01g L-serine-methylester hydrochloride in 130 ml of water were added at 0°C 29.8g of benzyl chloroformate. The pH was kept at 6-7 by adding a 40% aqueous solution of sodium hydroxide. After 3 h at 0°C the mixture was extracted with 5 portions of ethyl acetate, the combined extracts were dried and evaporated to give 37.12g (95%) of pure methyl 3-hydroxy-(S)-2-benzyloxycarbonylamino-propionate. IR (neat): 3380m (NH, OH), 1720s, br. (C=O), 1540s (amide II).

### Example 8

To a solution of 8.86g of methyl 3-hydroxy-(S)-2-benzyloxycarbonylamino-propionate and 4.00g of methanesulfonyl chloride in 60ml of ethyl acetate were added at -10°C 3.54g of triethylamine. After 1 hour at -10°C the mixture was washed with diluted aqueous hydrochloric acid (1N) and water, the ethylacetate was evaporated and the residue was triturated with hexane. The crude mesylate was dissolved in 11 ml of dimethylformamide and treated at - 20°C with a solution of 5.19g sodium thiophenolate in 22 ml of dimethylformamide. After 5 hours at -20°C the mixture was diluted with water and extracted with toluene. The toluene was evaporated and the residue was crystallized from toluene/hexane (1:9, v/v) to give 9.5g (79%) of pure methyl (3-phenylthio-(R)-2-benzyloxycarbonylamino)-propionate. IR (KBr): 3339m (NH), 1742s and 1681s (C=O), 1533s (amide II).

### Example 9

To a solution of 10.36g of methyl (3-phenylthio-(R)-2-benzyloxycarbonyl-amino)- propionate in 80ml of tetrahydrofuran were added at -80°C 18ml of a 1.67M solution of butyllithium in hexane followed by addition of 3.58g of chlorotrimethylsilane. The suspension was treated with 4.66g of bromochloromethane followed by addition of 27ml of a 1.67M solution of butyllithium in hexane at -80°C. The solution was quenched with 55ml of 6% aqueous hydrochloric acid and warmed to 22°C. The layers were separated, the organic layer was washed with brine, dried and evaporated to give 10.5g approx. 75% pure benzyl [3-chloro-2-oxo-(R)-1-(phenylthiomethyl)-propyl]-carbamate. IR (KBr): 3350s (NH), 1730s and 1685s (C=O), 1520s (amide II).

### Example 10

To a suspension of 2.15g aluminium isopropoxide in 35ml of isopropanol were added 3.64g of benzyl [3-chloro-2-oxo-(R)-1-(phenylthiomethyl)-propyl]-carbamate and the mixture was stirred at 50°C/ 400mbar for 4 hours. The mixture was cooled to 0°C, the pH was adjusted to 1 by adding hydrochloric acid and the isopropanol was evaporated. The residue was partitioned between water and dichloromethane and the organic layer was evaporated. The residue was chromatographed on silica to give 2.9g (80%) of pure benzyl (1R, 2S)-[3-chloro-2-hydroxy-1-(phenylthiomethyl)-propyl]-carbamate. IR (KBr): 3360br (NH, OH), 1690s (C=O), 1540s (amide II).

## Claims

1. A process for the manufacture of 2-[3(R)-amino-2-(R)-hydroxy-4-phenylthio-butyl]-N-tert.butyl-decahydro-(4aS, 8aS)-isoquinoline-3(S)-N-tert.-butyl-carboxamide of the formula 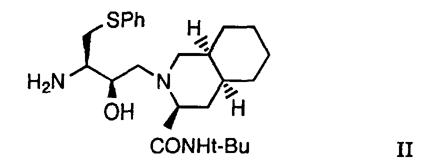 which process comprises
(a) reacting diprotected L-serine of formula 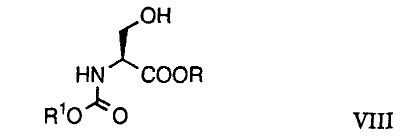 wherein R is C₁₋₆-alkyl and R¹ is C₁₋₆-alkyl or benzyl, with mesyl or tosyl chloride and a thiophenolate;
(b) reacting the resulting phenylthio compound of formula 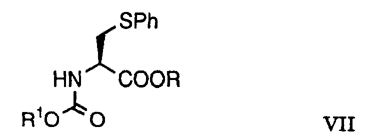 with a C₁₋₆-alkyl-lithium and an organochlorosilane of the formula ClSi(R²,R³,R⁴), wherein R²,R³ and R⁴ are C₁₋₆-alkyl or phenyl, and treating the silyl-protected compound of general formulae VII-A and/or VII-B formed as intermediate(s) 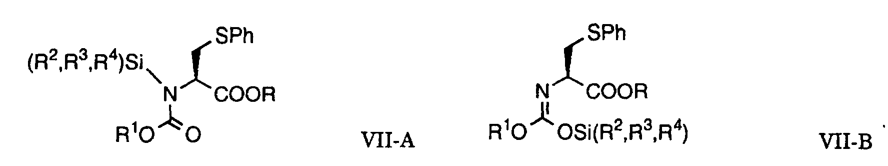 in the presence of dihalogenated methane with a C₁₋₆-alkyllithium;
(c) reducing a resulting halogen ketone of formula 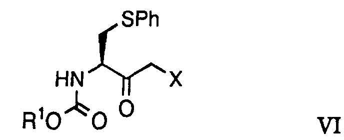 wherein X is halogen,
to the corresponding halogen alcohol of formula 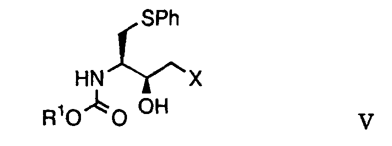
(d) treating the halogen alcohol of formula V with a base to form the [(R)-1-[(S)-oxiran-2-yl]-2-phenylthio-ethyl]-carbamic acid ester of formula 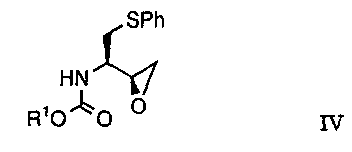
(e) reacting the carbamic acid ester of formula IV with N-tert.-butyl-decahydro-(4aS, 8aS)-isoquinoline-3(S)-carboxamide of formula 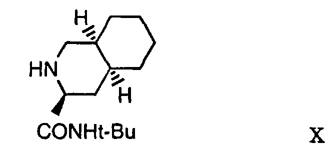 and
(f) treating the resulting compound of formula 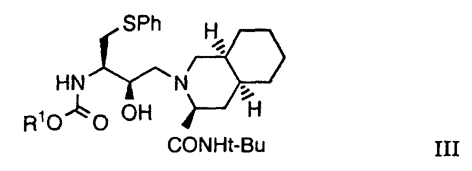 with a base.

2. The process of claim 1, wherein R and R¹ are methyl and X is chlorine.

3. The process of claim 1
**characterized by** the use of an aluminium trialkoxide or a lithium aluminium trialkoxyhydride as reducing agent in step c)

4. A compound of formula 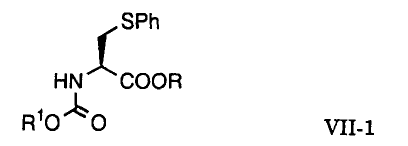 wherein R is C₁₋₆-alkyl and R¹ is C₁₋₆-alkyl.

5. A compound as claimed in claim 4 wherein R¹ is methyl or ethyl.

6. The compound of claim 4 which is methyl (3-phenylthio-(R)-2-methoxycarbonylamino)-propionate.

7. A compound of formula 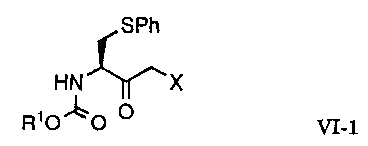 wherein R¹ is C₁₋₆-alkyl and X is halogen.

8. A compound as claimed in claim 7 wherein R¹ is methyl or ethyl.

9. The compound of claim 8 which is methyl [3-chloro-2-oxo-(R)-1-(phenylthiomethyl)-propyl]-carbamate.

10. A compound of formula 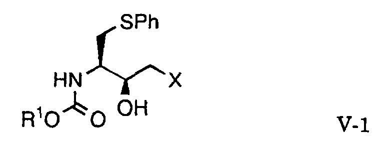 wherein R¹ is C₁₋₆-alkyl and X is halogen.

11. A compound as claimed in claim 10 wherein R¹ is methyl or ethyl.

12. The compound of claim 10 which is methyl (1R,2S)-[3-chloro-2-hydroxy-1-(phenylthiomethyl)-propyl]-carbamate.

13. A compound of formula 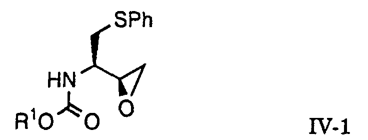 wherein R¹ is C₁₋₆-alkyl.

14. A compound as claimed in claim 13 wherein R¹ is methyl or ethyl.

15. The compound of claim 13 which is methyl [(R)-1-[(S)-oxiran-2-yl]-2-phenylthio-ethyl]-carbamate.

16. A compound of formula 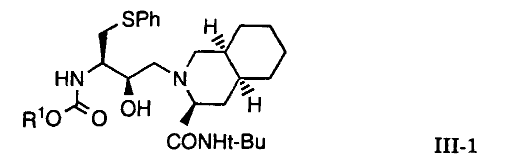 wherein R¹ is C₁₋₆-alkyl.

17. A compound as claimed in claim 16 wherein R¹ is methyl or ethyl.

18. The compound of claim 16 which is methyl (1R,2R)-[1-phenylthiomethyl-3-[3S,4aS,8aS)-3-tert.-butoxycarbamoyl-decahydro-isoquinol-2-yl]-2-hydroxypropyl]-carbamate.

## Patentansprüche

1. Verfahren zur Herstellung von 2-[3(R)-Amino-2-(R)-hydroxy-4-phenylthio-butyl]-N-tert-butyl-decahydro-(4aS,8aS)-isochinolin-3(S)-N-tert-butyl-carboxamid der Formel 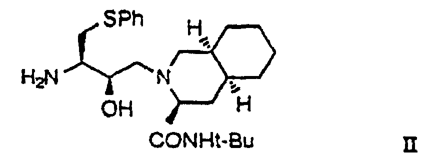 wobei das Verfahren umfasst
(a) Umsetzen von di-geschütztem L-Serin der Formel 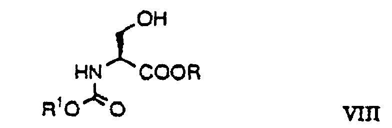 worin R C₁₋₆-Alkyl darstellt und R¹ C₁₋₆-Alkyl oder Benzyl darstellt, mit Mesyl- oder Tosylchlorid und einem Thiophenolat;
(b) Umsetzen der erhaltenen Phenylthioverbindung der Formel 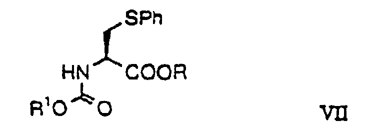 mit einem C₁₋₆-Alkyllithium und einem Organochlorsilan der Formel ClSi(R²,R³,R⁴), worin R², R³ und R⁴ C₁₋₆-Alkyl oder Phenyl darstellen, und Behandeln der Silyl-geschützten Verbindung der allgemeinen Formeln VII-A und/oder VII-B, gebildet als Zwischenprodukt(e) 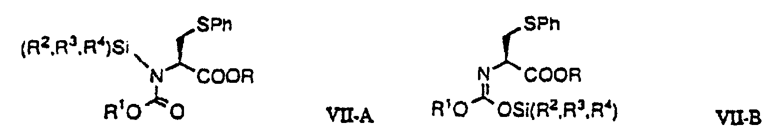 in Gegenwart von dihalogeniertem Methan mit einem C₁₋₆-Alkyllithium;
(c) Reduzieren des erhaltenen Halogenketons der Formel 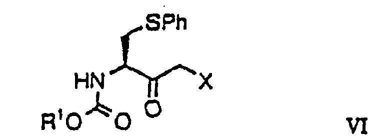 worin X Halogen darstellt,
zu dem entsprechenden Halogenalkohol der Formel 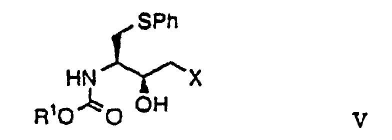
(d) Behandeln des Halogenalkohols der Formel V mit einer Base zur Bildung des [(R)-1-[(S)-Oxiran-2-yl]-2-phenylthio-ethyl]-carbaminsäureesters der Formel 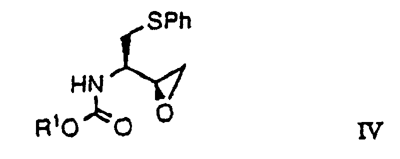
(e) Umsetzen des Carbaminsäureesters der Formel IV mit N-tert-Butyl-decahydro-(4aS,8aS)-isochinolin-3(S)-carboxamid der Formel 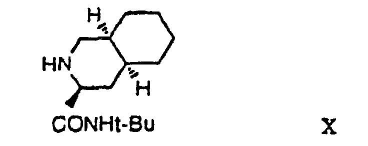 und
(f) Behandeln der erhaltenen Verbindung der Formel 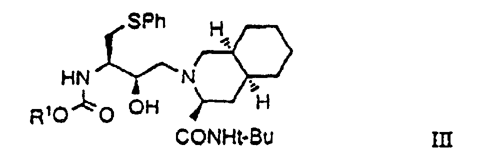 mit einer Base.

2. Verfahren nach Anspruch 1, worin R und R¹ Methyl darstellen und X Chlor darstellt.

3. Verfahren nach Anspruch 1,
**gekennzeichnet durch** die Verwendung eines Aluminiumtrialkoxids oder eines Lithiumaluminiumtrialkoxyhydrids als Reduktionsmittel in Schritt c).

4. Verbindung der Formel 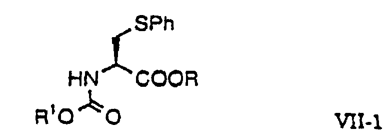 worin R C₁₋₆-Alkyl darstellt und R¹ C₁₋₆-Alkyl darstellt.

5. Verbindung nach Anspruch 4, worin R¹ Methyl oder Ethyl darstellt.

6. Verbindung nach Anspruch 4, nämlich (3-Phenylthio-(R)-2-methoxycarbonylamino)-propionsäuremethylester.

7. Verbindung der Formel 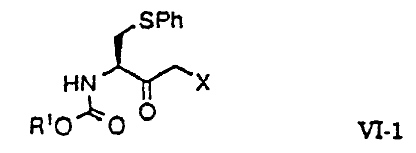 worin R¹ C₁₋₆-Alkyl darstellt und X Halogen darstellt.

8. Verbindung nach Anspruch 7, worin R¹ Methyl oder
Ethyl darstellt.

9. Verbindung nach Anspruch 8, nämlich [3-Chlor-2-oxo-(R)-1-(phenylthiomethyl)-propyl]-carbaminsäuremethylester.

10. Verbindung der Formel 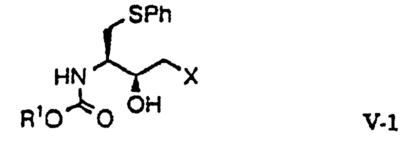 worin R¹ C₁₋₆-Alkyl darstellt und X Halogen darstellt.

11. Verbindung nach Anspruch 10, worin R¹ Methyl oder
Ethyl darstellt.

12. Verbindung nach Anspruch 10, nämlich (1R,2S)-[3-Chlor-2-hydroxy-1-(phenylthiomethyl)-propyl]-carbaminsäuremethylester.

13. Verbindung der Formel 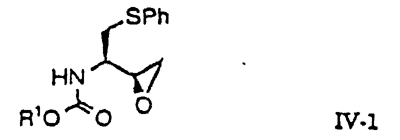 worin R¹ C₁₋₆-Alkyl darstellt.

14. Verbindung nach Anspruch 13, worin R¹ Methyl oder Ethyl darstellt.

15. Verbindung nach Anspruch 13, nämlich [(R)-1-[(S)-Oxiran-2-yl]-2-phenylthio-ethyl]-carbaminsäuremethylester.

16. Verbindung der Formel 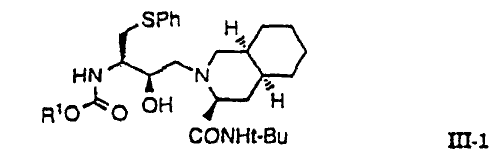 worin R¹ C₁₋₆-Alkyl darstellt.

17. Verbindung nach Anspruch 16, worin R¹ Methyl oder
Ethyl darstellt.

18. Verbindung nach Anspruch 16, nämlich (1R,2R)-[1-Phenylthiomethyl-3-(3S,4aS,8aS)-3-tert-butoxycarbamoyl-decahydro-isochinol-2-yl]-2-hydroxypropyl]-carbaminsäuremethylester.

## Revendications

1. Procédé de fabrication du 2-[3(R)-amino-2-(R)-hydroxy-4-phénylthio-butyl]-N-tert-butyl-décahydro-(4aS,8aS)-isoquinoléine-3(S)-N-tert-butyl-carboxamide de formule 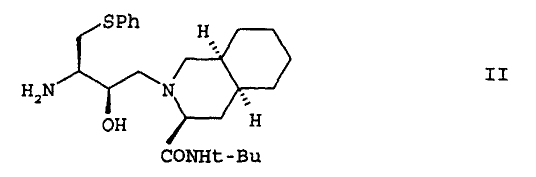 lequel procédé comprend les étapes suivantes :
(a) faire réagir la L-sérine di-protégée de formule 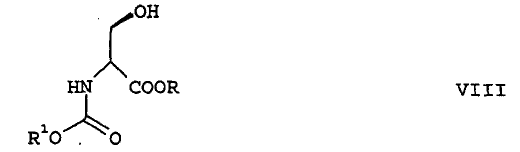 dans laquelle R est un groupe alkyle en C₁₋₆ et R¹ est un groupe alkyle en C₁₋₆ ou benzyle, avec un chlorure de tosyle ou de mésyle et un thiophénolate ;
(b) faire réagir le composé phénylthio résultant de formule 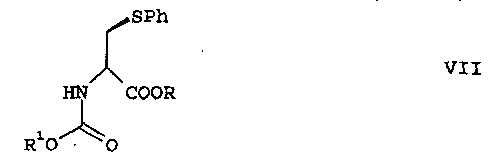 avec un alkyllithium en C₁₋₆ et un organochlorosilane de formule ClSi(R²,R³,R⁴), dans lequel R², R³ et R⁴ sont un groupe alkyle en C₁₋₆ ou phényle, et traiter le composé protégé par le groupe silyle de formule générale VII-A et/ou VII-B formé(s) comme intermédiaire(s) 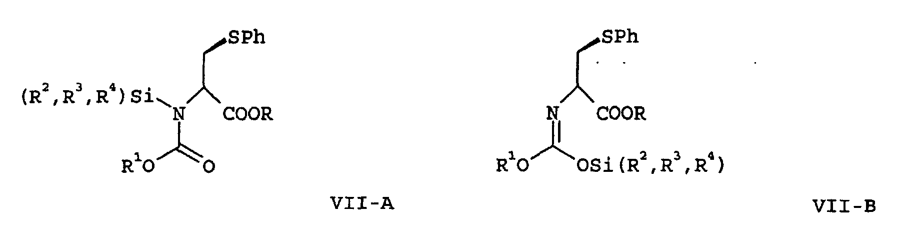 en présence de méthane dihalogéné avec un alkyllithium en C₁₋₆ ;
(c) réduire une cétone halogénée résultante de formule 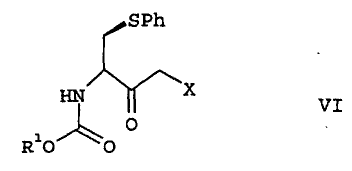 dans laquelle X est un halogène, en l'alcool halogéné correspondant de formule 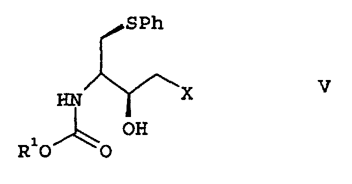
(d) traiter l'alcool halogéné de formule V avec une base pour former l'ester d'acide [(R)-1-[(S)-oxiran-2-yl]-2-phénylthioéthyl]carbamique de formule 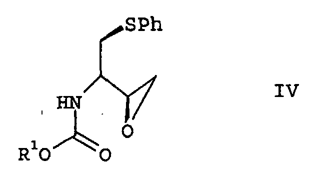
(e) faire réagir l'ester d'acide carbamique IV avec le N-tert-butyl-décahydro-(4aS, 8aS)-isoquinoléine-3(S)-carboxamide de formule 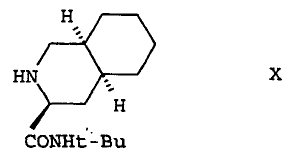 et
(f) traiter le composé résultant de formule 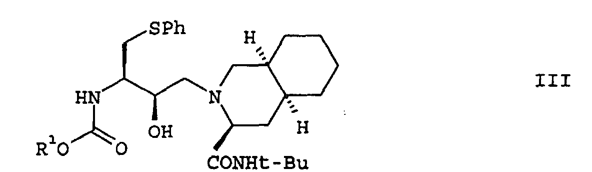 avec une base.

2. Procédé selon la revendication 1, dans lequel R et R¹ sont un groupe méthyle et X est du chlore.

3. Procédé selon la revendication 1,
**caractérisé par** l'utilisation d'un trialkoxyde d'aluminium ou d'un trialkoxyhydrure d'aluminium et de lithium comme agent réducteur dans l'étape c)

4. Composé de formule 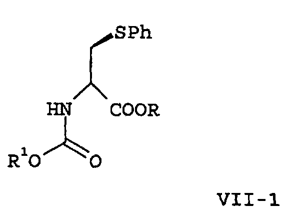 dans lequel R est un groupe alkyle en C₁₋₆ et R¹ est un groupe alkyle en C₁₋₆.

5. Composé selon la revendication 4 dans lequel R¹ est un groupe méthyle ou éthyle.

6. Composé selon la revendication 4 qui est le méthyl(3-phénylthio-(R)-2-méthoxycarbonylamino)propionate.

7. Composé de formule 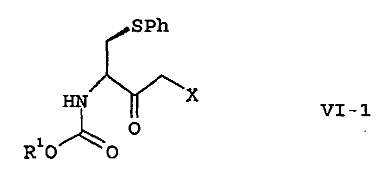 dans lequel R¹ est un groupe alkyle en C₁₋₆ et X est un halogène.

8. Composé selon la revendication 7 dans lequel R¹ est un groupe méthyle ou éthyle.

9. Composé selon la revendication 8 qui est le méthyl[3-chloro-2-oxo-(R)-1-(phénylthiométhyl)-propyl]carbamate.

10. Composé de formule 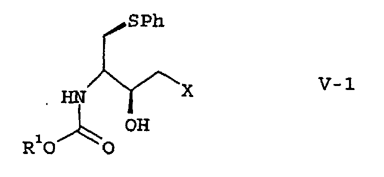 dans lequel R¹ est un groupe alkyle en C₁₋₆ et X est un halogène.

11. Composé selon la revendication 10 dans lequel R¹ est un groupe méthyle ou éthyle.

12. Composé selon la revendication 10 qui est le méthyl(1R,2S)-[3-chlro-2-hydroxy-1-(phénylthiométhyl)-propyl]carbamate.

13. Composé de formule 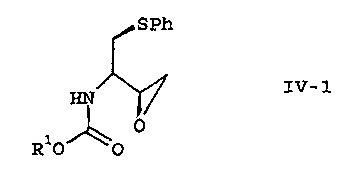 dans lequel R¹ est un groupe alkyle en C₁₋₆.

14. Composé selon la revendication 13 dans lequel R¹ est un groupe méthyle ou éthyle.

15. Composé selon la revendication 13 qui est le méthyl[(R)-1-[(S)-oxiran-2-yl]-2-phénylthioéthyl]-carbamate.

16. Composé de formule 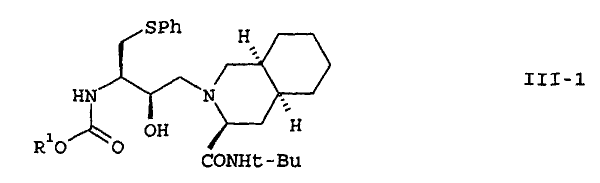 dans lequel R¹ est un groupe alkyle en C₁₋₆.

17. Composé selon la revendication 16 dans lequel R¹ est un groupe méthyle ou éthyle.

18. Composé selon la revendication 16 qui est le méthyl(1R,2S)-[1-phénylthiométhyl-3-[3S,4aS,8aS)-3-tert-butoxycarbamoyl-décahydro-isoquinol-2-yl]-2-hydroxy-propyl]carbamate.
